# FASCICULE DE BREVET EUROPEEN

(11) **EP 2 799 764 B1**
(45) Date de publication et mention de la délivrance du brevet: **08.07.2015**
(21) Numéro de dépôt: 14154474.2
(22) Date de dépôt: 10.02.2014
(51) Int. Cl.: F21V 7/00, F21V 13/00, F21W 131/205, F21Y 101/02, F21Y 111/00, F21Y 113/00

(54) **Dispositif d'éclairage pour former une tache d'éclairement à diamètre et température de couleur variables**
Beleuchtungsvorrichtung zur Bildung eines Lichtfleckdurchmessers und variabler Farbtemperatur
Lighting device for forming a luminous spot diameter and variable color temperature

(30) Priorité: 03.05.2013 FR 1354082
(43) Date de publication de la demande: 05.11.2014
(73) Titulaire: Maquet SAS, 45160 Ardon (FR)
(72) Inventeur: Valteau, Cécilia, 45240 Ligny le Ribault (FR); Vu Thi, Minh Hong, 45000 Orléans (FR)
(74) Mandataire: Prugneau, Philippe

(56) Documents cités:
- EP-A2- 2 299 163
- FR-A2- 2 339 129

## Description

### Domaine technique

L'invention concerne un dispositif d'éclairage, notamment pour l'éclairage d'un champ opératoire médical.

### Technique antérieure

En milieu médical, notamment en bloc opératoire, les conditions d'éclairage doivent être adaptées pour que l'utilisateur, par exemple un chirurgien ou un médecin, puisse travailler correctement. En particulier, l'éclairage doit être le plus homogène possible pour que l'utilisateur puisse distinguer les différents types de tissus biologiques dans la tache d'éclairement. En outre, la lumière, globalement blanche, doit se conformer à certaines normes et présenter un indice de rendu des couleurs (IRC) compris entre 85 et 100, ainsi qu'une température de couleurs comprise entre 3000 degrés Kelvin (K) et 6700 K. Par ailleurs, il est souvent souhaitable que l'utilisateur puisse faire varier certaines caractéristiques spectrales de la lumière, dont la température de couleur, pour l'adapter à son besoin. Idéalement, une telle variation des caractéristiques spectrales ne doit pas être accompagnée de variation de l'éclairement visuel. Enfin, il est souvent utile d'avoir une tache d'éclairement de dimension variable.

Actuellement, il existe différents types de dispositifs d'éclairage répondant en partie à ces exigences, qui mélangent la lumière issue de plusieurs sources de lumières pour obtenir une lumière d'éclairage blanche.

On connaît par exemple du document de brevet EP 2 299 163 un dispositif d'éclairage comportant deux séries de LEDs (Light Emitting Diode), blanches, de températures de couleurs distinctes respectivement, à savoir blanche chaude et blanche froide, réparties en alternance sur la périphérie d'un réflecteur central qui focalise la lumière émise par ces LEDs pour former la tache d'éclairement. La température de couleur résultante de ce dispositif d'éclairage peut être modifiée. Toutefois, le volume de lumière résultant n'est pas homogène. De plus, quand un utilisateur regarde le dispositif d'éclairage, il voit des alternances de températures de couleur ce qui est visuellement inconfortable.

On connaît aussi du document de brevet US 7,465,065 un dispositif d'éclairage à LEDs blanches et colorées juxtaposées pour obtenir globalement une lumière blanche. Toutefois, un tel dispositif d'éclairage produit une lumière non homogène. De plus, lorsqu'un obstacle masque une partie du flux lumineux, par exemple lorsque l'utilisateur se penche sous le dispositif d'éclairage, l'équilibre entre les contributions des différentes LEDs est rompu, ce qui modifie la température de couleur et provoque un effet d'irisation conduisant à la formation d'ombres colorées dans la tache d'éclairement. De plus, ce dispositif d'éclairage ne permet pas de faire varier les dimensions de la tache d'éclairement.

On connaît aussi du document de brevet DE 10 2006 040 393 un dispositif d'éclairage comportant des LEDs blanches et colorées couplées ensemble à une optique de focalisation unique permettant d'obtenir une lumière blanche de température de couleur ajustable. Pour améliorer le mélange des couleurs, un guide de lumière est interposé entre les LEDs et une optique de focalisation. Cependant, un tel guide de lumière réduit le rendement optique du dispositif d'éclairage d'où une puissance électrique consommée élevée.

On connaît des documents de brevet US 2008/174868 et US 2006/164726 des dispositifs pour projeter de la lumière sur un écran avec des LEDs colorées couplées à une optique. Cependant ces systèmes utilisant des LEDs de couleurs, une température de couleur ne peut leur être attribuée. De plus ces dispositifs ne semblent pas permettre de modification de la dimension de la zone d'éclairement.

### Exposé de l'invention

Le but de l'invention est donc de remédier à ces inconvénients en proposant un dispositif d'éclairage offrant un éclairement homogène, un rendement optique élevé, sans créer d'ombres colorées dans le champ d'éclairage et autorisant une variation de la dimension de la tache d'éclairement et aussi une variation de la température de couleur de l'éclairage.

A cet effet, l'invention a pour objet un dispositif d'éclairage pour l'éclairage d'un champ opératoire comprenant une première source de lumière apte à émettre un premier faisceau de lumière à une première température de couleur et une seconde source de lumière apte à émettre un second faisceau de lumière à une seconde température de couleur différente de la première température de couleur et un système optique pour, à partir de la lumière des sources, former une tache d'éclairement à une température de couleur intermédiaire comprise entre la première température de couleur et la seconde température de couleur, caractérisé en ce que le système optique comprend un diviseur de faisceau agencé pour diviser la lumière produite par chaque source de lumière en un faisceau de lumière transmis et un faisceau de lumière réfléchi, en ce que le système optique est agencé pour focaliser ensemble la seconde partie de faisceau de lumière réfléchi dudit faisceau de lumière avec la première partie de faisceau de lumière transmis dudit second faisceau pour former un premier faisceau résultant ayant ladite température de couleur intermédiaire et ensemble la première partie du faisceau de lumière transmis dudit premier faisceau avec la seconde partie du faisceau de lumière réfléchi dudit second faisceau pour former un second faisceau résultant ayant ladite même température de couleur intermédiaire et les combiner suivant une certaine configuration de superposition dans un plan de superposition où est formée ladite tache d'éclairement à une température de couleur intermédiaire, et en ce que le système optique avec les sources de lumière sont agencés pour modifier la dimension de la tache d'éclairement à la dite température intermédiaire.

De manière conventionnelle, le diamètre D10 d'une tache d'éclairement globalement circulaire correspond au diamètre de la tache d'éclairement à 10% de l'éclairement maximal de la même tache d'éclairement mesuré à un mètre de l'éclairage.

Selon un mode de réalisation préférentiel de l'invention, la dimension de la tache d'éclairement peut varier entre un petit diamètre D10, par exemple 10cm, et un grand diamètre D10, par exemple 20cm, et la variation du diamètre de la tache d'éclairement s'effectue sans aucun mouvement mécanique dans le dispositif d'éclairage.

Selon d'autres modes de réalisation de l'invention, la variation du diamètre de la tache d'éclairement peut être obtenu par un déplacement relatif des sources de lumière par rapport au diviseur de faisceau dans le dispositif d'éclairage, ou encore par un déplacement relatif du système optique par rapport au diviseur de faisceau dans le dispositif d'éclairage, ou avec une combinaison de différents déplacements relatifs des sources et du système optique par rapport au diviseur de faisceau.

La variation de la température de couleur de la tache d'éclairement est obtenue en pilotant l'intensité des courants d'alimentation dans les sources de lumière.

Le dispositif d'éclairage selon l'invention peut ainsi avantageusement présenter les particularités suivantes :
- lesdites sources de lumière peuvent être des LEDs blanches;
- lesdites sources de lumière peuvent comprendre des premières LEDs disposées à une première distance du diviseur de faisceau et des secondes LEDs disposées à une seconde distance du diviseur de faisceau qui est différente de ladite première distance; les LEDS à la première distance du diviseur de faisceau servent à former une petite tache d'éclairement tandis que les LEDs à la seconde distance du diviseur de faisceau servent à former une plus grande tache d'éclairement; avec cet agencement, on peut moduler le diamètre de la tache d'éclairement sans mouvement mécanique dans le dispositif d'éclairage et en particulier avec des sources lumineuses qui sont statiques par rapport au diviseur de faisceau ce qui simplifie la construction du dispositif d'éclairage tout en gardant l'éclairement constant par un pilotage de l'intensité des courants d'alimentation dans les sources lumineuses;
- lesdites premières LEDs disposées à la première distance du diviseur de faisceau comprennent une première couronne de LEDs ayant ladite première température de couleur et une seconde couronne de LEDs ayant ladite seconde température de couleur, et lesdites secondes LEDs disposées à la seconde distance du diviseur de faisceau comprennent une troisième couronne de LEDs ayant ladite première température de couleur et une quatrième couronne de LEDs ayant ladite seconde température de couleur, lesdites première et troisième couronnes de LEDs étant décalées l'une de l'autre suivant un axe d'éclairement du dispositif d'éclairage et lesdites seconde et quatrième couronnes de LEDs étant décalées l'une de l'autre suivant une direction radiale par rapport à cet axe d'éclairement; cet agencement contribue à l'obtention d'une tache d'éclairement centrale à un axe d'éclairement d'une coupole d'éclairage avec possibilité de variation du diamètre de la tache sans aucun mouvement mécanique dans l'éclairage;

- les LEDs de la première et de la troisième couronne de LEDs sont disposées en alternance les unes par rapport aux autres en définissant une sorte de dentelure suivant l'axe d'éclairement et les LEDs de la seconde et de la quatrième couronne de LEDs sont disposées en alternance les unes par rapport aux autres en définissant une sorte de dentelure suivant la direction radiale; cet agencement des LEDs permet un montage compact des LEDs dans une coupole d'éclairage ;
- une alimentation électrique est prévue pour alimenter en courants les LEDs et moduler les courants pour provoquer une modification de la dimension de la tache d'éclairement et/ou une modification de la température de couleur de la tache d'éclairement;
- l'alimentation électrique est agencée en outre pour moduler lesdits courants de telle façon à former une tache d'éclairement à un niveau d'éclairement constant;
- lesdites sources de lumière peuvent aussi comprendre des LEDs montées mobiles par rapport au diviseur de faisceau; un tel agencement permet aussi de modifier la dimension de la tache d'éclairement ;
- le système optique comprend un réflecteur elliptique et une lentille pour focaliser lesdits faisceaux de lumière transmis et réfléchis et ce réflecteur elliptique peut être monté mobile par rapport au diviseur de faisceau et/ou cette lentille peut être montée mobile par rapport au diviseur de faisceau ce permet aussi de modifier la dimension de la tache d'éclairement;
- le réflecteur elliptique peut être un réflecteur à facettes ce qui permet d'augmenter la profondeur du champ d'éclairage et aussi les performances de dilution des ombres de la coupole;
- le système optique peut comprendre en outre un miroir disposé pour dévier à 90° la lumière produite par une source delumière vers le diviseur de faisceau ce qui contribue à la compacité du dispositif d'éclairage; des lentilles peuvent aussi être prévues devant chaque LED pour collecter un maximum de flux lumineux issue de la LED dans un angle solide plus petit;
- le diviseur de faisceaux peut être un miroir semi-réfléchissant orienté à 45° par rapport aux sources de lumière ou LEDs.

### Description sommaire des dessins

La présente invention sera mieux comprise et d'autres avantages apparaîtront à la lecture de la description détaillée de plusieurs modes de réalisation pris à titre d'exemples nullement limitatifs et illustrés par les dessins annexés dans lesquels :
- la figure 1 est une vue en perspective d'un dispositif d'éclairage selon l'invention utilisé dans un bloc opératoire ;
- la figure 2 est une représentation schématique du montage du diviseur de faisceau ;
- la figure 3 est une vue en perspective schématique du système optique d'un dispositif d'éclairage selon l'invention ;
- la figure 4A est une représentation schématique du système optique et des sources de lumière pour former une « petite tache d'éclairement » ;
- la figure 4B est une représentation schématique du système optique et des sources de lumière pour former une « grande tache d'éclairement » ;
- la figure 5 est une représentation schématique des sources de lumière et du système optique selon une disposition compacte du dispositif d'éclairage selon l'invention ;
- les figures 6 et 7 illustrent la disposition particulière des LEDs pour avoir une variation de la dimension de la tache d'éclairement sans mouvement mécanique ;
- les figures 8 à 11 illustrent la disposition des LEDs mobiles dans le dispositif d'éclairage selon l'invention pour faire varier la dimension de la tache d'éclairement ;
- les figures 12 et 13 illustrent un dispositif d'éclairage selon l'invention avec un système optique à lentille mobile ;
- la figure 14 illustre un dispositif d'éclairage selon l'invention avec un système optique à réflecteur mobile ;
- la figure 15 illustre un dispositif d'éclairage selon l'invention avec un réflecteur à facettes ;
- les figures 16 à 18 sont des graphiques illustrant le profil de l'éclairement visuel normalisé en fonction de la distance à l'axe central de la coupole pour le module d'éclairage de la figure 4A en configuration « petite tache » (courbes en pointillés courts) et le module d'éclairage de la figure 4B en configuration « grande tache » (courbes en pointillés longs) avec la tache d'éclairement résultante (courbe en trait continu), en fonction du diamètre en mm de la tache d'éclairement ;
- la figure 19 est un graphique illustrant, sur une échelle de 0 à 1, l'intensité de l'éclairement visuel avec le dispositif d'éclairage des figures 8 à 11 en configurations « petite et grande taches » (courbes en trait continu) et dans une configuration « intermédiaire » (trait continu), en fonction du diamètre en mm de la tache d'éclairement ;
- la figure 20 est un graphique illustrant, pour différents réglages en pourcentage (%) des intensités des premier et second courants alimentant les LEDs, la variation de la température de couleur résultante en K (Kelvin).

### Description d'un exemple de réalisation

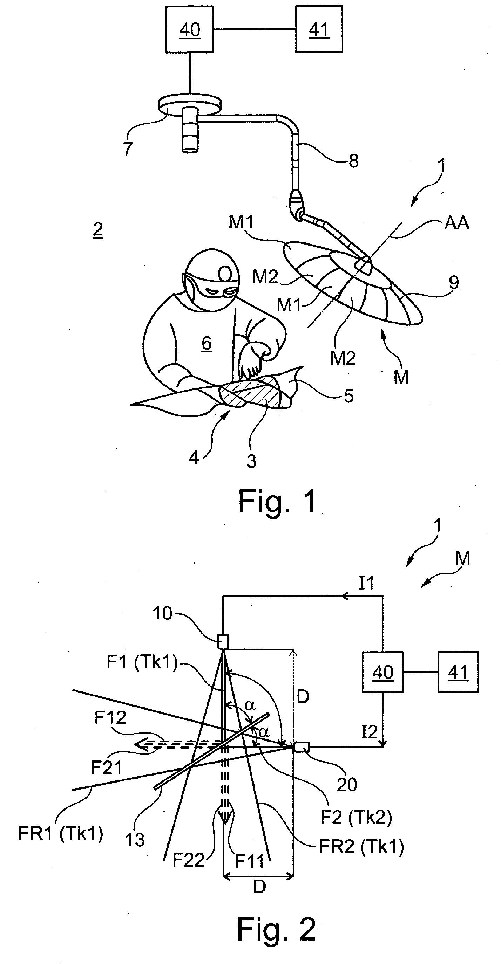

En référence à la figure 1, le dispositif d'éclairage 1 selon l'invention est en particulier destiné à être utilisé dans un bloc opératoire 2 pour former une tache d'éclairement 3 (schématisée par des hachures) dans un champ opératoire 4, par exemple sur le corps d'un patient 5 opéré par un chirurgien 6. Dans cet exemple et de manière connue, le dispositif d'éclairage 1 comporte une embase 7 fixée au plafond du bloc opératoire 2 et de laquelle s'étend un bras articulé 8 portant une coupole d'éclairage 9 dans laquelle sont disposés par exemple une pluralité de modules d'éclairage M pourvus ici de LEDs (non visibles sur cette figure) qui se présentent chacun ici sous la forme d'un quartier de la coupole pour fournir une tache d'éclairement 3 centrée dans l'axe d'éclairement AA de la coupole.

Le dispositif d'éclairage 1 selon l'invention est prévu pour former une tache d'éclairement 3 à dimension et à température de couleur variables à partir des modules de lumière M, chaque module de lumière comportant deux sources de lumière ayant des température de couleur différentes, par exemple deux LEDs blanches (ou diode électroluminescente) disposées symétriquement par rapport à un diviseur de faisceau. Plus particulièrement, cette tache d'éclairement est formée par la superposition des éclairages centrés sur l'axe AA produits par les différents modules de lumière M.

La figure 2 illustre le principe de la division des faisceaux lumineux produits par deux LEDs 10, 20 dans un module de lumière M. La première LED 10 est apte à émettre un premier faisceau F1 de lumière (schématisé par un double trait continu) ayant une première température de couleur Tk1. La seconde LED 20 est apte à émettre un second faisceau F2 de lumière (schématisé par un trait simple continu) ayant une seconde température de couleur Tk2 différente de la première température de couleur Tk1.

On utilise de préférence des première et seconde LEDs 10, 20 géométriquement identiques présentant des températures de couleur Tk1, Tk2 différentes, provenant du même fournisseur, ayant un même boîtier, des mêmes puces électroniques et nécessitant un même type d'alimentation. On peut ainsi limiter les effets de toute différence de flux lumineux entre les premières, secondes LEDs 10, 20 pour garantir un éclairage homogène.

Pour obtenir un éclairage adapté au milieu médical, on choisira des LEDs 10, 20 blanches ayant un indice de rendu des couleurs élevé, compris entre 85 et 100, de préférence compris entre 90 et 100, voire compris entre 95 et 100 et une température de couleur Tk1, Tk2 comprise entre 3000 K et 5000 K. A titre d'exemple, on peut utiliser une LED 10 ayant une première température de couleur Tk1 de 3000 K et une LED 20 ayant une seconde température de couleur Tk2 de 5000 K. Ainsi, lorsque les LEDs 10, 20 sont traversées respectivement par des premier et second courants électriques I1, I2 sensiblement identiques en intensité, si l'on superpose les premier et second faisceaux F1, F2, on obtient un éclairage en lumière blanche ayant une température de couleur intermédiaire Tkr d'environ 4000 K.

Sur la figure 2, les première et seconde LEDs 10, 20 sont orientées de sorte que les axes médians des faisceaux F1, F2 soient orientés à 90° l'un par rapport à l'autre et on comprend donc que ces LEDs sont disposées symétriquement par rapport au diviseur de faisceau 13.

Le diviseur de faisceau 13 est par exemple un miroir dichroïque ou semi-réfléchissant à haute efficacité, neutre sur le plan spectral et comprenant une lame support (en verre ou en matière synthétique) recouverte de plusieurs couches ayant des traitements d'optique. Le diviseur de faisceau 13 est apte à diviser chacun des premier et second faisceau F1, F2 en une première partie de faisceau F11, F21 (schématisées respectivement par un double trait continu et par un trait simple continu) transmis par le diviseur de faisceau 13, et en une seconde partie de faisceau F12, F22 (schématisées respectivement par un double trait pointillé et par un trait simple pointillé) réfléchis par le diviseur de faisceau 13. On utilise de préférence un diviseur de faisceau 13 permettant de diviser chaque faisceau F1, F2 avec un rendement théorique de 100%, c'est-à-dire sans perte, dont par exemple 50% en réflexion et 50% en transmission ou encore par exemple 30% en réflexion et 70% en transmission.

Le diviseur de faisceau 13 est disposé à égale distance D des LEDs 10, 20 et forme un même angle α de 45° avec chacun des faisceaux F1, F2. De plus, le diviseur de faisceau 13 et les LEDs 10, 20 sont agencés spatialement de sorte que le premier faisceau F1 et le second faisceau F2 atteignent le diviseur de faisceau 13, à l'opposé l'un de l'autre, de part et d'autre du diviseur de faisceau 13. Ainsi, la seconde partie F12 du premier faisceau F1 se superpose ou se combine à ladite première partie F21 du second faisceau F2 pour former un premier faisceau résultant FR1 ayant une température de couleur intermédiaire résultante Tkr comprise entre les première et seconde températures de couleur Tk1, Tk2. De plus, la première partie F11 du premier faisceau F1 se superpose ou se combine à la seconde partie F22 du second faisceau F2 pour former un second faisceau résultant FR2 ayant une même température de couleur résultante Tkr.

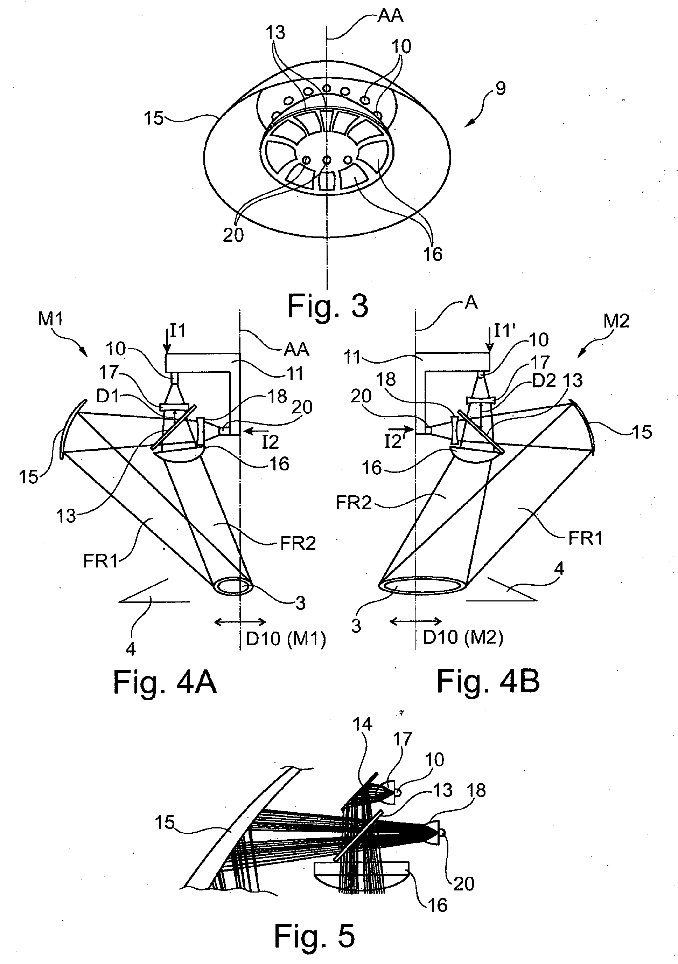

Bien que non représenté sur la figure 2 (mais visible sur la figure 4), chaque LED 10,20 est équipée d'une lentille qui sert à collimater la lumière produite par la LED sur le diviseur 13, les ensembles LED 10 avec sa lentille et LED 20 avec sa lentille étant symétriques par rapport au diviseur 13 et placés respectivement à égale distance D de celui-ci.

Le module d'éclairage M permet donc de produire à une certaine température de couleur Tkr un flux lumineux total égal à la somme des flux lumineux respectifs des première et seconde LEDs 10, 20.

Les LEDs 10,20 de chaque module d'éclairage M sont reliées électriquement à une alimentation électrique 40 couplée à une unité de contrôle/commande 41 (UC) apte à piloter l'alimentation électrique 40 pour alimenter la première LED 10 avec une premier courant électrique I1 et alimenter la seconde LED 20 avec un second courant électrique I2 qui peut être différent de I1 ce qui permet de faire varier la température de couleur.

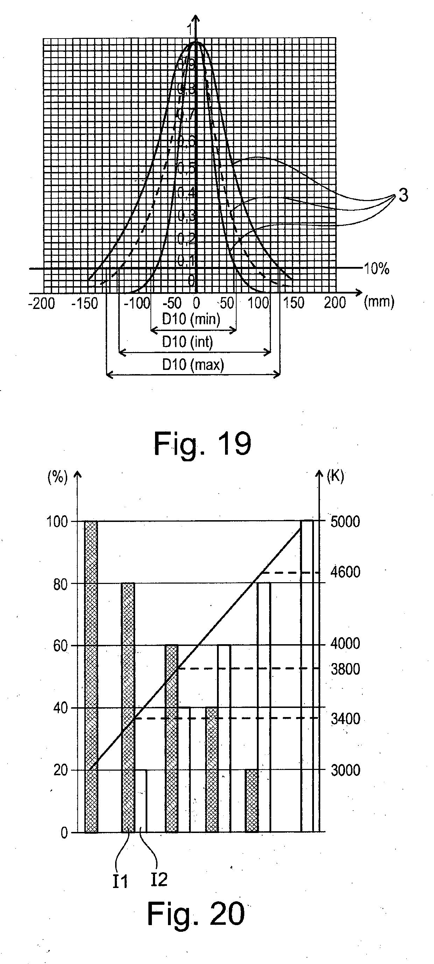

L'alimentation électrique 40 peut se présenter sous la forme d'une alimentation électrique unique pour toutes les LEDs des modules M ou sous la forme de deux alimentations électriques distinctes alimentant sélectivement respectivement toutes les LEDs 10 et toutes les LEDs 20. Il est connu que le flux lumineux d'une LED dépend de l'intensité du courant qui la traverse. Pour réaliser la modulation de température de couleur, l'alimentation électrique 40 est pilotée par l'UC 41 de sorte à moduler l'intensité des premier et second courants électriques I1, I2 selon le principe des vases communicants, comme illustré par le graphique de la figure 20, c'est-à-dire de façon sensiblement complémentaire et opposée.

Sur la figure 20, on a illustré six configurations d'alimentation distinctes en courants électriques I1, I2. Pour une première configuration d'alimentation, on alimente dans un module M la LED 10 avec 100% du courant électrique I1 et la LED 20 avec 0% du courant I2. Ainsi, seule la LED 10 est utilisée et la température de couleur résultante Tkr correspond à la première température de couleur Tk1 de la LED 10, dans cet exemple de 3000 K. Pour une seconde configuration d'alimentation, on alimente la LED 10 avec 80% du courant électrique I1 et la LED 20 avec 20% du courant I2. La température de couleur résultante Tkr correspondante est comprise entre les première et seconde températures de couleur Tk1, Tk2, dans cet exemple de 3400 K. Les courants I1, I2 peuvent encore être modulés jusqu'à atteindre une sixième configuration d'alimentation selon laquelle on alimente la LED 10 avec 0% du courant électrique I1 et la LED 20 avec 100% du courant I2. La température de couleur résultante Tkr correspond à la température de couleur Tk2 de la LED 10, dans cet exemple de 5000 K.

Ainsi, avec un module de lumière M, on peut former une tache d'éclairement 3 avec une température de couleur variable comprise entre Tk1 et Tk2.

Dans la coupole 9, on comprend qu'il y a plusieurs modules de lumière M répartis autour de l'axe d'éclairement AA de sorte que les LEDs 10 des modules de lumière M forment une première couronne autour de l'axe d'éclairement AA et les LEDs 20 de ces modules de lumière M forment une seconde couronne autour de l'axe AA coaxiale à la première couronne de LEDs 10. La première couronne de LEDs 10 a un diamètre supérieur à celui de la seconde couronne de LEDs 20. On comprend aussi qu'on a donc dans la coupole 9 une série de diviseurs de faisceau 13 répartis autour de l'axe d'éclairement AA et qui forment aussi une couronne de diviseurs de faisceau coaxiale aux couronnes de LEDs 10,20 comme visible sur la figure 3.

Selon l'invention, on utilise le principe de construction du module de lumière M exposé ci-dessus pour réaliser un dispositif d'éclairage 1 notamment en forme de coupole avec lequel on peut former en plus une tache d'éclairement 3 de dimension variable dans le plan de travail qui correspond au champ opératoire 4 distant d'environ 1 m de la coupole.

Selon un mode de réalisation préférentiel, on utilise des premiers modules de lumière M1 à LEDs 10,20 dédiés à la formation d'une petite tache d'éclairement de par exemple 10 cm de diamètre et des seconds modules de lumière M2 à LEDs 10, 20 dédiés à la formation d'une plus grande tache d'éclairement de par exemple 20 cm de diamètre, et une alimentation électrique qui ajuste les courants d'alimentation des LEDs dans les modules de lumière pour modifier la dimension de la tache d'éclairement 3.

Les figures 4A et 4B représentent de façon très schématique respectivement un module de lumière M1 à LEDs 10,20 dédié à la formation d'une petite tache d'éclairement 3 centrée sur l'axe d'éclairement AA de la coupole et un module de lumière M2 à LEDs 10,20 dédié à la formation d'une grande tache d'éclairement 3 centrée sur l'axe d'éclairement AA.

Dans le module de lumière M1 de la figure 4A, les LEDs 10,20 sont placées à une distance D1 du diviseur 13. Dans le module de lumière M2 de la figure 4B, les LEDs 10,20 sont placées à une distance D2 du diviseur 13, D2 étant différente de D1 et ici plus grande que D1 (mais on pourrait aussi avoir D2 plus petite que D1 pour optimiser l'encombrement de la coupole). La référence 11 désigne un support sur lequel sont fixées les LEDs 10,20 dans la coupole 9.

On a aussi représenté sur les figures 4A et 4B, chaque LED 10 avec une lentille 17 pour collimater le faisceau de lumière F1 sur le diviseur 13, et aussi chaque LED 20 avec une lentille 18 pour collimater le faisceau de lumière F2 sur le diviseur 13. Ces lentilles 17, 18 sont identiques et placées à la même distance devant les LEDs 10,20 respectivement. Elles sont par exemple des lentilles biconcaves aptes à diminuer la divergence des faisceaux de lumière F1, F2 ce qui contribue à la compacité de la coupole d'éclairage 9. A noter qu'il faut que ces lentilles soient toujours placées à la même distance des LEDs respectives.

Comme encore illustré sur les figures 4A et 4B, chaque module de lumière M1, M2 a un système optique qui comprend un réflecteur 15 et une lentille 16, ici un réflecteur elliptique, lesquels à partir des faisceaux de lumière incidents F12,F21,F11,F22 (montrés sur la figure 2) servent à former la tache d'éclairement 3 dans le plan de travail.

Sur la figure 5, on a illustré un agencement compact du dispositif d'éclairage 1 selon l'invention, dans lequel dans chaque module M, un miroir 14 est interposé entre le diviseur de faisceau 13 et la lentille de la LED 10 pour forcer le faisceau F1 à faire un angle droit. Avec cet agencement spatial, on peut gagner de la place en hauteur dans la coupole 9 et ainsi réduire l'encombrement ce celle-ci.

Dans le module de lumière M1 dédié à la formation d'une petite tache d'éclairement 3, on a l'image virtuelle de la LED 10 (du fait de la lentille 17) qui correspond au foyer objet de la lentille 16 et l'image virtuelle de la LED 20 (du fait de la lentille 18) qui correspond au foyer objet du réflecteur 15. L'agencement du module de lumière M1 est donc apte à produire une petite tache d'éclairement 3 dans le plan de travail par la combinaison d'une focalisation (étranglement de faisceau) et une superposition des faisceaux de lumière FR1 et FR2 issus du réflecteur 15 et de la lentille 16.

Maintenant comme cela apparaît sur la figure 4B, du fait de la distance D2 différente de D1, on n'a plus cette correspondance image virtuelle/foyer dans le module de lumière M2 mais une image virtuelle de la LED 10 décalée du foyer objet de la lentille 16 et une image virtuelle de la LED 20 décalée du foyer objet du réflecteur 15. L'agencement du module de lumière M2 est donc apte à produire une grande tache d'éclairement 3 (c'est à dire une tache plus grande que la tache produite par le module M1) dans le plan de travail par la combinaison d'une défocalisation (élargissement de faisceau) et d'une superposition des faisceaux de lumière FR1 et FR2 issus du réflecteur 15 et de la lentille 16.

Dans un dispositif d'éclairage selon l'invention en forme de coupole d'éclairage, on a en pratique les modules M1 et M2 disposés en alternance autour de l'axe d'éclairement AA. On a dans la coupole une série de réflecteurs 15 des modules M1 et M2 répartis autour de l'axe d'éclairement AA et formant une sorte de couronne coaxiale aux couronnes de LEDs 10,20 et une série de lentilles 16 de ces modules M1 et M2 réparties également autour de l'axe d'éclairement AA et formant une autre couronne coaxiale aux couronnes de LEDs comme illustré sur la figure 3.

Au moyen de l'UC 41, on pilote l'alimentation électrique 40 de sorte à envoyer sélectivement des courants I1, I2 respectivement dans les LEDs 10, 20 des modules de lumière M1 et des courants I1' et I2' respectivement dans les LEDs 10, 20 des modules de lumière M2 pour modifier la dimension de la tache d'éclairement et/ou modifier la température de couleur comme exposé ci-dessus.

Il faut comprendre que dans cet agencement, pour garder l'homogénéité de température de couleur dans la tache d'éclairement 3 à dimension variable, chaque module de lumière M1 et M2 doit envoyer la même température de couleur. En d'autres termes, le ratio I1/I2 doit être identique au ratio I1'/I2' pour obtenir une tache d'éclairement 3 à la température de couleur intermédiaire Tkr et on fait varier ce ratio pour faire varier la température de couleur dans la tache d'éclairement entre Tk1 et Tk2. Pour faire varier le diamètre de la tache d'éclairement, on fait varier le courant relatif entre les modules de lumière M1 et M2, c'est à dire le ratio I1/I1' et donc I2/I2'.

Pour une température de couleur intermédiaire Tkr fixe entre Tk1 et Tk2, on peut faire varier I1' et I1 ou I2' et I2 selon le principe des vases communicants pour faire varier le diamètre D10 de la tache d'éclairement entre un grand diamètre (le diamètre de la tache produite par les modules de lumière M2 seuls) et un petit diamètre (le diamètre de la tache produite par les modules de lumière M1 seuls). A noter qu'il faut que la somme des courants I1+I1' ou I2+I2' soit sensiblement constante pour avoir une variation de diamètre de tache à éclairement constant, tout en conservant le ratio I1/I2 égal à I1'/I2' pour conserver la même température de couleur dans la tache d'éclairement 3.

Plus particulièrement, si les courants I1' et I2' sont nuls, la tâche d'éclairement 3 est produite que par les modules de lumière M1 seuls et on a donc une petite tache d'éclairement avec un diamètre de par exemple 10cm.

Si les courants I1 et I2 sont nuls, la tache d'éclairement 3 est produite que par les modules de lumière M2 seuls et on a donc une grande tache d'éclairement avec un diamètre de par exemple 20 cm.

Si on fournit des courants non nuls I1 et I2 aux modules de lumière M1 et des courants non nuls I1' et I2' aux modules de lumière M2, on produit une tache d'éclairement avec un diamètre intermédiaire entre 10 et 20 cm.

Pour garder un même niveau d'éclairement pour les différents diamètres de tache d'éclairement, l'UC 41 ajuste le courant dans toutes les LEDs des modules de lumière M1 et M2 tout en gardant un ratio I1'/I2' identique au ratio I1/I2 de façon à ne pas changer la température de couleur pour la tache d'éclairement 3. Si on passe d'une tache d'éclairement d'un certain diamètre à une tache d'éclairement plus grande, l'UC 41 doit augmenter les courants avec la même proportion pour chaque LED.

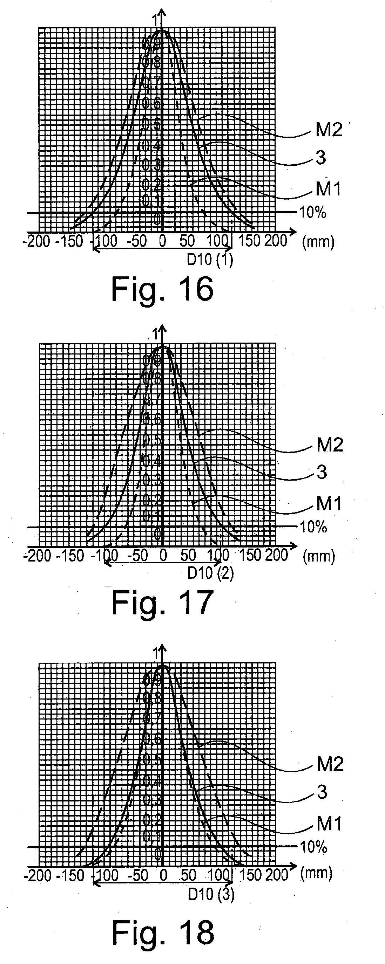

Sur les graphes des figures 16 à 18, l'éclairement produit par les modules M1 seuls en configuration « petite tache » est tracé en pointillés courts. L'éclairement produit par les modules M2 seuls en configuration « grande tache » est tracé en pointillés longs. Le tracé en trait continu représente la superposition des éclairements produits par les modules M1 et M2.

On voit sur ces figures 16 à 18 comment la dimension de la tache d'éclairement 3 varie en fonction des ratios I1/I1' et I2/I2', ici avec des ratios 20/80, 50/50 et 80/20. Ces ratios peuvent être pilotés par l'UC 41 de telle façon à maintenir une puissance d'éclairage constante quand le diamètre de la tache d'éclairement est changé par le chirurgien.

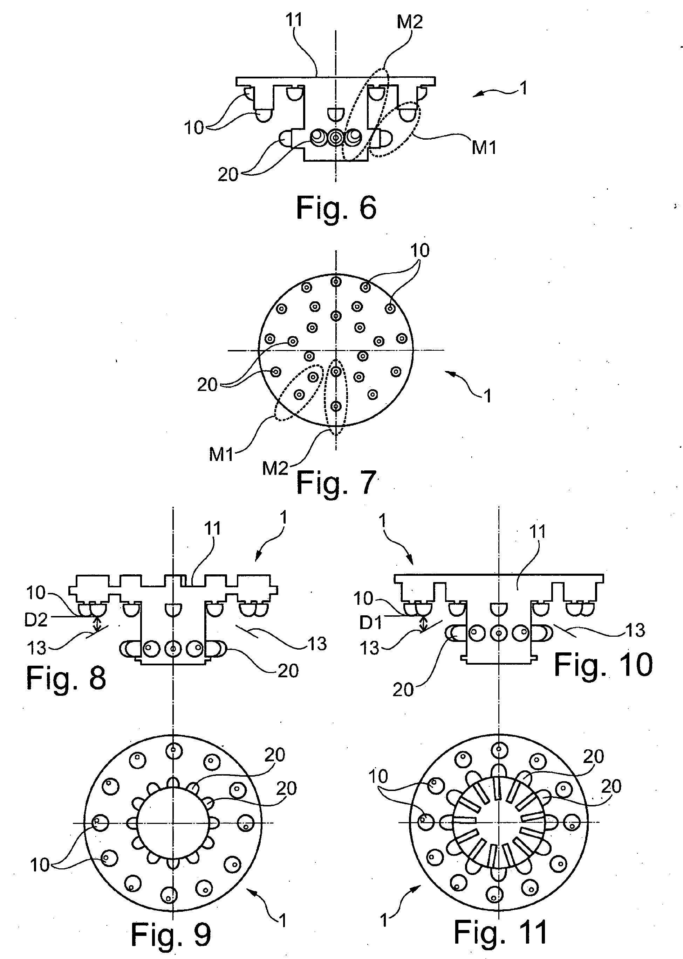

Sur les figures 6 et 7, on voit la disposition relative des LEDs 10 et 20 des modules M1 et M2 sur le support 11. On voit que les LEDs 10 des modules M1 sont disposées en alternance avec les LEDs 10 des modules M2 suivant la périphérie des deux couronnes de LEDs 10 ce qui fait une sorte de dentelure de LEDs suivant la direction axiale de la coupole et que les LEDs 20 des modules M1 sont disposées en alternance avec les LEDs 20 des modules M2 suivant la périphérie des deux couronnes de LEDs 20 ce qui fait une autre sorte de dentelure de LEDs suivant une direction radiale perpendiculaire à cette direction axiale.

Dans la coupole 9, les LEDs 10 des modules M1 et M2 forment deux couronnes de LEDs coaxiales entre elles suivant l'axe d'éclairage central AA, les LEDs 10 d'une couronne étant décalées axialement des LEDs 10 de l'autre couronne suivant cet axe d'éclairage central. Les LEDs 20 des modules M1 et M2 forment deux autres couronnes de LEDs coaxiales entre elles suivant l'axe d'éclairage AA mais les LEDs 20 d'une couronne sont décalées radialement des LEDs 20 de l'autre couronne.

On a donc dans la coupole 9, deux couronnes de LEDs 10 et 20 qui sont placées symétriquement par rapport à la couronne de diviseurs de faisceau 13 et à la distance D1 de celle-ci, ces deux couronnes de LEDs étant dédiées à la formation d'une petite tache d'éclairement et deux autres couronnes de LEDs 10 et 20 qui sont placées symétriquement par rapport à la même couronne de diviseurs de faisceau 13 et à la distance D2 de celle-ci, ces deux autres couronnes de LEDs étant dédiées à la formation de la grande tache d'éclairement. Dans ce mode de réalisation préférentiel, on peut modifier la dimension et la température de couleur de la tache d'éclairement sans mouvement mécanique dans la coupole ce qui simplifie sa fabrication et augmente sa fiabilité.

Selon un autre mode de réalisation du dispositif d'éclairage selon l'invention, les LEDs 10,20 dans un module de lumière M sont montées mobiles par rapport au système optique et en particulier par rapport au diviseur de faisceaux 13 pour modifier la dimension de la tache d'éclairement 3. Dans ce mode de réalisation, la coupole comporte qu'une seule couronne de LEDs 10 (avec lentille 17) et qu'une seule couronne de LEDs 20 (avec lentille 18), les deux couronnes de LEDs étant liées en mouvement sur le support 11 pour s'approcher ou s'éloigner ensembles du diviseur de faisceaux 13.

Sur les figures 8 et 9, on a illustré les deux couronnes de LEDs 10 et 20 mobiles sur le support 11 dans la coupole. Sur ces figures, les LEDs 10,20 sont espacées des diviseurs de faisceaux 13 d'une grande distance D2 pour former une grande tache d'éclairement 3.

Sur les figures 10 et 11, on a représenté les mêmes LEDs 10, 20 qui ont été rapprochées pour être espacées des diviseurs de faisceaux 13 de la plus petite distance D1 pour former une petite tache d'éclairement 3. Il est entendu que les lentilles 17 et 18 se déplacent avec les LEDs 10 et 20.

Le support 11 peut se présenter sous la forme d'un disque portant les LEDS 10 et d'un tube coaxial portant les LEDs 20. Le déplacement relatif des LEDS 10 et 20 peut être obtenu par exemple par un coulissement télescopique du tube support.

En relation avec les figures 8 à 11, on peut prévoir un agencement dans lequel les LEDs 20 sont en plus mobiles radialement et les LEDs 10 sont en plus mobiles axialement. On peut donc avoir un double déplacement en translation de chaque LED 10 et 20 (avec leur lentille 17,18) ce qui permet un réglage fin des distances D1 et D2 et donc du diamètre de la tache d'éclairement 3.

On comprend qu'avec un déplacement relatif des LEDs 10 et 20 par rapport au diviseur 13, on défocalise les faisceaux lumineux FR1 et FR2 produits par les LEDs 10 et 20 et il en résulte une variation de la dimension de la tache d'éclairement 3. Sur le graphique de la figure 19, on a illustré encore différentes taches d'éclairement 3 correspondant à différentes positions des LEDs 10 et 20 par rapport au diviseur 13.

On comprend que dans les modes de réalisation du dispositif d'éclairage suivant les figures 6 à 11, le diviseur de faisceaux 13 peut rester fixe dans la coupole. Il est entendu ici que l'UC 41 pilote les courants d'alimentation des LEDs 10,20 pour maintenir un éclairement à puissance constante quelque soit la position spatiale des LEDs.

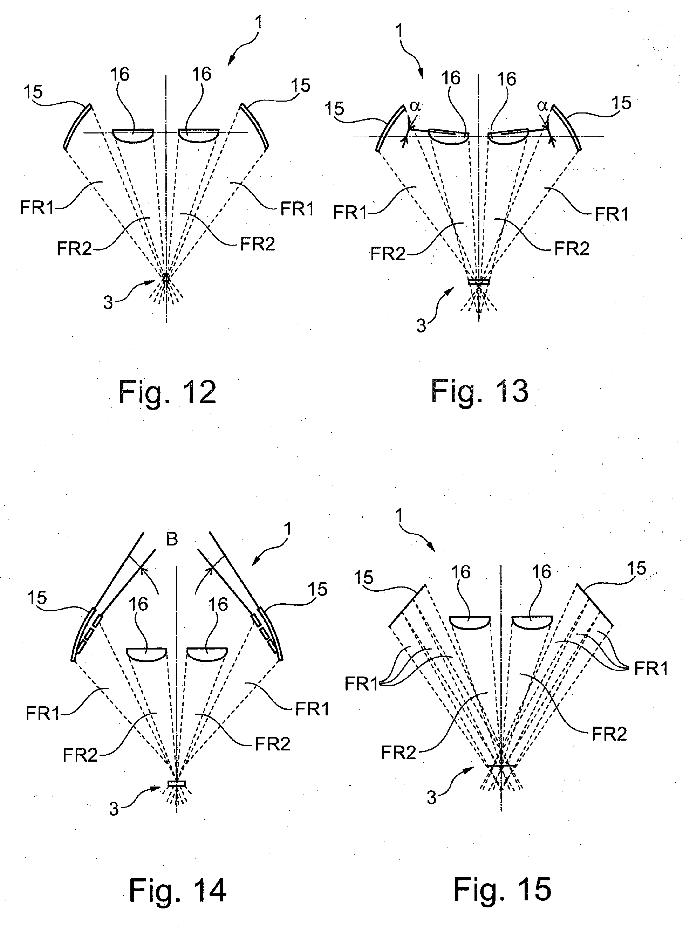

Sur les figures 12 et 13, on a illustré encore un autre mode de réalisation du dispositif d'éclairage pour former une tache d'éclairement à dimension variable. Dans ce mode de réalisation, on utilise une lentille 16 montée mobile en rotation dans la coupole pour pivoter d'un angle α entre une première position où les faisceaux FR2 sont focalisés et se superposent avec les faisceaux FR1 pour former une petite tache d'éclairement (figure 12) et une seconde position où les faisceaux FR2 focalisés sont décalés dans le plan de superposition ce qui change la configuration de superposition des faisceaux FR1 et FR2 dans le plan de superposition pour former une grande tache d'éclairement (figure 13). On peut comprendre que les positions angulaires intermédiaires de la lentille 16 entre la première et la seconde position correspondent à autant de dimensions différentes de la tache d'éclairement 3.

Sur la figure 14, on a illustré encore un autre mode de réalisation du dispositif d'éclairage pour former une tache d'éclairement 3 à dimension variable. Selon cette configuration, le réflecteur 15 est monté mobile en rotation dans la coupole pour pivoter d'un angle β entre une première position angulaire (en trait pointillé) où les faisceaux FR1 sont focalisés et se superposent avec les faisceaux FR2 pour former une petite tache d'éclairement 3 et une seconde position angulaire (en trait plein) où les faisceaux FR1 focalisés sont décalés ce qui change la configuration de superposition des faisceaux FR1 et FR2 dans le plan de superposition pour former une grande tache d'éclairement 3. On peut comprendre que les positions intermédiaires du réflecteur 15 entre la première et la seconde position correspondent à autant de dimensions différentes de la tache d'éclairement 3.

Sur la figure 15, on a représenté un réflecteur elliptique 15 à facettes avec des facettes agencées pour diviser le faisceau FR1 en plusieurs petits faisceaux focalisés à différentes altitudes dans l'axe central d'éclairement AA de la coupole 9. Ce réflecteur 15 peut aussi être monté mobile comme indiqué plus haut dans la coupole. Un réflecteur 15 à facettes permet d'augmenter la profondeur du champ d'éclairage.

Le dispositif d'éclairage selon l'invention comporte donc des sources de lumière, un système optique et une alimentation en courants des sources de lumière qui sont agencés pour placer les faisceaux lumineux issus des sources selon différentes configurations de superposition des faisceaux lumineux dans le plan de superposition qui correspond au plan de travail où est formée la tache d'éclairement, ces différentes configurations correspondant à différentes dimensions ou différents diamètres de la tache d'éclairement.

Il va de soi que la présente invention ne saurait être limitée à la description qui précède de certains modes de réalisation, susceptibles de subir quelques modifications sans pour autant sortir du cadre de l'invention. Ainsi, par exemple, la tache d'éclairement 3 n'est pas forcément circulaire et peut être par exemple ovale.

Par ailleurs, les différents modes de réalisation de l'invention présentés ci-dessus peuvent être combinés sans sortir du cadre de l'invention. On pourrait ainsi avoir dans la coupole des LEDs fixes et/ou mobiles avec un réflecteur et/ou une lentille mobile.

## Revendications

1. Dispositif d'éclairage pour l'éclairage d'un champ opératoire comprenant une première source de lumière (10) apte à émettre un premier faisceau (F1) de lumière à une première température de couleur (Tk1) et une seconde source de lumière (20) apte à émettre un second faisceau (F2) de lumière à une seconde température de couleur (Tk2) différente de la première température de couleur et un système optique pour, à partir de la lumière des sources, former une tache d'éclairement (3) à une température de couleur intermédiaire (Tkr) comprise entre la première température de couleur et la seconde température de couleur, **caractérisé en ce que** le système optique comprend un diviseur de faisceau (13) agencé pour diviser la lumière produite par chaque source de lumière (10,20) en un faisceau de lumière transmis (F11,F21) et un faisceau de lumière réfléchi (F12,F22), **en ce que** le système optique est agencé pour focaliser ensemble la seconde partie de faisceau de lumière réfléchi (F12) dudit faisceau de lumière (F1) avec la première partie de faisceau de lumière transmis (F21) dudit second faisceau pour former un premier faisceau résultant (FR1) ayant ladite température de couleur intermédiaire (Tkr) et ensemble la première partie du faisceau de lumière transmis (F11) dudit premier faisceau (F1) avec la seconde partie du faisceau de lumière réfléchi (F22) dudit second faisceau (F2) pour former un second faisceau résultant (FR2) ayant ladite même température de couleur intermédiaire (Tkr) et les combiner suivant une certaine configuration de superposition dans un plan de superposition où est formée ladite tache d'éclairement à une température de couleur intermédiaire, et **en ce que** le système optique avec les sources de lumière sont agencés pour modifier la dimension de la tache d'éclairement (3) à ladite température intermédiaire.

2. Dispositif d'éclairage selon la revendication 1, **caractérisé en ce que** lesdites sources de lumière comprennent des premières LEDs (10,20) disposées à une première distance (D1) du diviseur de faisceau (13) et des secondes LEDs (10,20) disposées à une seconde distance (D2) du diviseur de faisceau (13) qui est différente de ladite première distance.

3. Dispositif d'éclairage selon la revendication 2, **caractérisé en ce que** lesdites premières LEDs disposées à la première distance (D1) du diviseur de faisceau (13) comprennent une première couronne de LEDs (10) ayant ladite première température de couleur (Tk1) et une seconde couronne de LEDs (20) ayant ladite seconde température de couleur (Tk2), et **en ce que** lesdites secondes LEDs disposées à la seconde distance (D2) du diviseur de faisceau (13) comprennent une troisième couronne de LEDs (10) ayant ladite première température de couleur (Tk1) et une quatrième couronne de LEDs (20) ayant ladite seconde température de couleur (Tk2), **en ce que** lesdites première et troisième couronnes de LEDs sont décalées l'une de l'autre suivant un axe d'éclairement du dispositif d'éclairage et **en ce que** lesdites seconde et quatrième couronnes de LEDs sont décalées l'une de l'autre suivant une direction radiale par rapport à cet axe d'éclairement.

4. Dispositif d'éclairage selon la revendication 3, **caractérisé en ce que** les LEDs (10) de la première et de la troisième couronne de LEDs sont disposées en alternance les unes par rapport aux autres en définissant une sorte de dentelure suivant l'axe d'éclairement et **en ce que** les LEDs (20) de la seconde et de la quatrième couronne de LEDs sont disposées en alternance les unes par rapport aux autres en définissant une sorte de dentelure suivant la direction radiale.

5. Dispositif d'éclairage selon l'une des revendications 2, 4 ou 5, **caractérisé en ce qu'**il comporte une alimentation électrique (40) pour alimenter en courants (I1,I2,I1',I2') les LEDs (10,20) et moduler les courants pour provoquer une modification de la dimension de la tache d'éclairement (3) et/ou une modification de la température de couleur de la tache d'éclairement (3).

6. Dispositif d'éclairage selon la revendication 5, **caractérisé en ce que** l'alimentation électrique (40) est agencée pour moduler lesdits courants de telle façon à former une tache d'éclairement à un niveau d'éclairement constant.

7. Dispositif d'éclairage selon l'une des revendications précédentes, **caractérisé en ce que** lesdites sources de lumière comprennent des LEDs (10,20) montées mobiles par rapport au diviseur de faisceau (13).

8. Dispositif d'éclairage selon l'une des revendications précédentes, **caractérisé en ce que** ledit diviseur de faisceaux (13) est un miroir semi-réfléchissant orienté à 45° par rapport auxdites sources de lumière.

9. Dispositif d'éclairage selon l'une des revendications précédentes, **caractérisé en ce que** le système optique comprend un réflecteur elliptique (15) et une lentille (16) pour focaliser lesdits faisceaux de lumière transmis et réfléchis.

10. Dispositif d'éclairage selon la revendication 9, **caractérisé en ce que** ledit réflecteur elliptique (15) est monté mobile par rapport au diviseur de faisceau.

11. Dispositif d'éclairage selon la revendication 9 ou 10, **caractérisé en ce que** ledit réflecteur elliptique (15) est un réflecteur à facettes.

12. Dispositif d'éclairage selon la revendication 9, **caractérisé en ce que** ladite lentille (16) est montée mobile par rapport au diviseur de faisceau.

13. Dispositif d'éclairage selon l'une des revendications précédents, **caractérisé en ce que** le système optique comprend en outre un miroir (14) disposé pour dévier à 90° la lumière produite par une source de lumière vers le diviseur de faisceau (13).

14. Dispositif d'éclairage selon l'une des revendications précédentes, **caractérisé en ce que** lesdites sources de lumières (10,20) sont des LEDs blanches.

## Patentansprüche

1. Beleuchtungseinrichtung zur Beleuchtung eines Operationsbereichs, umfassend eine erste Lichtquelle (10), die in der Lage ist, einen ersten Lichtstrahl (F1) mit einer ersten Farbtemperatur (Tk1) auszusenden, und eine zweite Lichtquelle (20), die in der Lage ist, einen zweiten Lichtstrahl (F2) mit einer von der ersten Farbtemperatur verschiedenen zweiten Farbtemperatur (Tk2) auszusenden, und ein optisches System, um ausgehend von dem Licht der Quellen einen Beleuchtungsfleck (3) mit einer zwischen der ersten Farbtemperatur und der zweiten Farbtemperatur umfassten intermediären Farbtemperatur (Tkr) zu bilden,
**dadurch gekennzeichnet,**
**dass** das optische System einen Strahlteiler (13) enthält, der ausgebildet ist zum Teilen des von jeder Lichtquelle (10, 20) erzeugten Lichts in einen transmittierten Lichtstrahl (F11, F21) und einen reflektierten Lichtstrahl (F12, F22),
**dass** das optische System ausgebildet ist zum gemeinsamen Fokussieren des zweiten Teils des reflektierten Lichtstrahls (F12) des Lichtstrahls (F1) mit dem ersten Teil des transmittierten Lichtstrahls (F21) des zweiten Lichtstrahls zum Bilden eines ersten resultierenden Strahls (FR1) mit der intermediären Farbtemperatur (Tkr) und zum gemeinsamen Fokussieren des ersten Teils des transmittierten Lichtstrahls (F11) des ersten Lichtstrahls (F1) mit dem zweiten Teil des reflektierten Lichtstrahls (F22) des zweiten Strahls (F2) zum Bilden eines zweiten resultierenden Strahls (FR2) mit derselben intermediären Farbtemperatur (Tkr) und um diese gemäß einer bestimmten Überlagerungskonfiguration in einer Überlagerungsebene zu kombinieren, in der der Beleuchtungsfleck bei einer intermediären Farbtemperatur gebildet ist,
und
**dass** das optische System und die Lichtquellen ausgebildet sind zum Ändern der Dimension des Beleuchtungsflecks (3) mit der intermediären Temperatur.

2. Beleuchtungseinrichtung nach Anspruch 1, **dadurch gekennzeichnet, dass** die Lichtquellen in einem ersten Abstand (D1) zu dem Strahlteiler (13) angeordnete erste LEDs (10, 20) und in einem von dem ersten Abstand verschiedenen zweiten Abstand (D2) zu dem Strahlteiler (13) angeordnete zweite LEDs (10, 20) umfassen.

3. Beleuchtungseinrichtung nach Anspruch 2, **dadurch gekennzeichnet, dass** die in dem ersten Abstand (D1) zu dem Strahlteiler (13) angeordneten ersten LEDs einen ersten Kranz von LEDs (10) mit der ersten Farbtemperatur (Tk1) und einen zweiten Kranz von LEDs (20) mit der zweiten Farbtemperatur (Tk2) umfassen, und dass die in dem zweiten Abstand (D2) zu dem Strahlteiler (13) angeordneten zweiten LEDs einen dritten Kranz von LEDs (10) mit der ersten Farbtemperatur (Tk1) und einen vierten Kranz von LEDs (20) mit der zweiten Farbtemperatur (Tk2) umfassen, dass die ersten und dritten Kränze von LEDs zueinander gemäß einer Beleuchtungsachse der Beleuchtungseinrichtung versetzt sind, und dass die zweiten und vierten Kränze von LEDs zueinander gemäß einer radialen Richtung in Bezug auf die Beleuchtungsachse versetzt sind.

4. Beleuchtungseinrichtung nach Anspruch 3, **dadurch gekennzeichnet, dass** die LEDs (10) des ersten und des dritten Kranzes von LEDs alternierend in Bezug zueinander angeordnet sind und eine Art Zahnung gemäß der Beleuchtungsachse definieren, und dass die LEDs (20) des zweiten und des vierten Kranzes von LEDs jeweils alternierend zueinander angeordnet sind und eine Art Zahnung gemäß der radialen Richtung definieren.

5. Beleuchtungseinrichtung nach einem der Ansprüche 2, 4 oder 5, **dadurch gekennzeichnet, dass** sie eine elektrische Versorgung (40) umfasst zum Versorgen der LEDs (10, 20) mit Strömen (I1, I2, I1', I2') und zum Modulieren der Ströme zum Bewirken einer Änderung der Dimension des Beleuchtungsflecks (3) und/oder einer Änderung der Farbtemperatur des Beleuchtungsflecks (3).

6. Beleuchtungseinrichtung nach Anspruch 5, **dadurch gekennzeichnet, dass** die elektrische Versorgung (40) ausgebildet ist zum Modulieren der Ströme derart, dass ein Beleuchtungsfleck bei konstantem Beleuchtungsniveau gebildet ist.

7. Beleuchtungseinrichtung nach einem der vorhergehenden Ansprüche, **dadurch gekennzeichnet, dass** die Lichtquellen bezüglich des Strahlteilers (13) beweglich montierte LEDs (10, 20) umfassen.

8. Beleuchtungseinrichtung nach einem der vorhergehenden Ansprüche, **dadurch gekennzeichnet, dass** der Strahlteiler (13) ein halbreflektierender Spiegel ist, der um 45° in Bezug auf die Lichtquellen orientiert ist.

9. Beleuchtungseinrichtung nach einem der vorhergehenden Ansprüche, **dadurch gekennzeichnet, dass** das optische System einen elliptischen Reflektor (15) und eine Linse (16) zum Fokussieren der transmittierten und reflektierten Lichtstrahlen umfasst.

10. Beleuchtungseinrichtung nach Anspruch 9, **dadurch gekennzeichnet, dass** der elliptische Reflektor (15) beweglich in Bezug auf den Strahlteiler montiert ist.

11. Beleuchtungseinrichtung nach Anspruch 9 oder 10, **dadurch gekennzeichnet, dass** der elliptische Reflektor (15) ein Reflektor mit Facetten ist.

12. Beleuchtungseinrichtung nach Anspruch 9, **dadurch gekennzeichnet, dass** die Linse (16) beweglich in Bezug auf den Strahlteiler montiert ist.

13. Beleuchtungseinrichtung nach einem der vorhergehenden Ansprüche, **dadurch gekennzeichnet, dass** das optische System ferner einen Spiegel (14) umfasst, der angeordnet ist, um das durch eine Lichtquelle erzeugte Licht um 90° in Richtung auf den Strahlteiler (13) umzulenken.

14. Beleuchtungseinrichtung nach einem der vorhergehenden Ansprüche, **dadurch gekennzeichnet, dass** die Lichtquellen (10, 20) weiße LEDs sind.

## Claims

1. A lighting device for illuminating an operative field, the lighting device comprising a first light source (10) suitable for emitting a first light beam (F1) at a first color temperature (Tk1), a second light source (20) suitable for emitting a second light beam (F2) at a second color temperature (Tk2) that is different from the first color temperature, and an optical system that uses the light from the sources to form an illumination spot (3) at an intermediate color temperature (Tkr) lying between the first color temperature and the second color temperature, said lighting device being **characterized in that** the optical system comprises a beam splitter (13) that is arranged to split the light produced by each light source (10, 20) into a transmitted light beam (F11, F21) and a reflected light beam (F12, F22), **in that** the optical system is arranged to focus the reflected second light beam portion (F12) of said first light beam (F1) together with the transmitted first light beam portion (F21) of said second light beam (F2) so as to form a first resulting beam (FR1) having said intermediate color temperature (Tkr), and to focus the transmitted first light beam portion (F11) of said first light beam (F1) together with the reflected second light beam portion (F22) of said second light beam (F2) so as to form a resulting second beam (FR2) having the same said intermediate color temperature (Tkr), and to combine them in a certain superposition configuration in a superposition plane in which said illumination spot is formed at an intermediate color temperature, and **in that** the optical system and the light sources are arranged to modify the size of the illumination spot (3) at said intermediate temperature.

2. A lighting device according to claim 1, **characterized in that** said light sources comprise first LEDs (10, 20) that are disposed at a first distance (D1) from the beam splitter (13) and second LEDs (10, 20) that are disposed at a second distance (D2) from the beam splitter (13) that is different from said first distance.

3. A lighting device according to claim 2, **characterized in that** said first LEDs disposed at the first distance (D1) from the beam splitter (13) comprise a first ring of LEDs (10) having said first color temperature (Tk1) and a second ring of LEDs (20) having said second color temperature (Tk2), **in that** said second LEDs disposed at the second distance (D2) from the beam splitter (13) comprise a third ring of LEDs (10) having said first color temperature (Tk1) and a fourth ring of LEDs (20) having said second color temperature (Tk2), **in that** said first and third rings of LEDs are offset from each other along an illumination axis of the lighting device, and **in that** said second and fourth rings of LEDs are offset from each other along a direction that is radial relative to said illumination axis.

4. A lighting device according to claim 3, **characterized in that** the LEDs (10) of the first and the third rings of LEDs are disposed in alternation relative to one another by defining a sort of crenellation along the illumination axis, and **in that** the LEDs (20) of the second and fourth ring of LEDs are disposed in alternation relative to one another by defining a sort of crenellation in the radial direction.

5. A lighting device according to any one of claims 2, 4, or 5, **characterized in that** it further comprises an electrical power supply (40) for feeding currents (I1, I2, I1', I2') to the LEDs (10, 20), and for modulating the currents so as to cause a modification in the size of the illumination spot (3), and/or a modification in the color temperature of the illumination spot (3).

6. A lighting device according to claim 5, **characterized in that** the electrical power supply (40) is arranged to modulate said currents in such a manner as to form an illumination spot at a constant illumination level.

7. A lighting device according to any preceding claim, **characterized in that** said light sources comprise LEDs (10, 20) mounted to move relative to the beam splitter (13).

8. A lighting device according to any preceding claim, **characterized in that** said beam splitter (13) is a semi-reflective mirror that is angularly positioned at 45° relative to said light sources.

9. A lighting device according to any preceding claim, **characterized in that** the optical system comprises an elliptical reflector (15) and a lens (16) for focusing said transmitted and reflected light beams.

10. A lighting device according to claim 9, **characterized in that** said elliptical reflector (15) is mounted to move relative to the beam splitter.

11. A lighting device according to claim 9 or claim 10, **characterized in that** said elliptical reflector (15) is a facetted reflector.

12. A lighting device according to claim 9, **characterized in that** said lens (16) is mounted to move relative to the beam splitter.

13. A lighting device according to any preceding claim, **characterized in that** the optical system further comprises a mirror (14) disposed to deflect the light produced by a light source through 90° towards the beam splitter (13).

14. A lighting device according to any preceding claim, **characterized in that** said light sources (10, 20) are white LEDs.
